# EUROPÄISCHE PATENTSCHRIFT

(11) **EP 2 516 421 B1**
(45) Veröffentlichungstag und Bekanntmachung des Hinweises auf die Patenterteilung: **10.08.2016**
(21) Anmeldenummer: 10795688.0
(22) Anmeldetag: 20.12.2010
(51) Int. Cl.: C07D 405/12, C07D 417/12

(54) **Neues Verfahren zur Herstellung von 4-Aminobut-2-enoliden ausgehend von 4-Alkoxyfuran-2(5H)-on oder 4-Arylalkoxyfuran-2-(5H)-on**
New method of producing 4-aminobut-2-enolides starting from 4-alkoxyfuran-2(5H)-one or 4-arylalkoxyfuran-2-(5H)-one
Nouveau procédé de fabrication de 4-aminobut-2-énolides à partir de 4-alkoxyfuran-2(5H)-one ou de 4-arylalkoxyfuran-2(5H)-one

(30) Priorität: 23.12.2009 EP 09180603; 28.12.2009 US 290385 P
(43) Veröffentlichungstag der Anmeldung: 31.10.2012
(73) Patentinhaber: Bayer Intellectual Property GmbH, 40789 Monheim (DE)
(72) Erfinder: LUI, Norbert, 51519 Odenthal (DE); HEINRICH, Jens-Dietmar, 51381 Leverkusen (DE)
(74) Vertreter: BIP Patents
(86) Internationale Anmeldenummer: PCT/EP2010/070202
(87) Internationale Veröffentlichungsnummer: WO 2011/076715

(56) Entgegenhaltungen:
- EP-A1- 2 042 496
- WO-A1-2007/115644
- SHANDALA M Y ET AL: "Reaction of methyl tetronate with some amines. Synthesis of substituted 4-aminobut-2-enolides", JOURNAL OF HETEROCYCLIC CHEMISTRY, WILEY-BLACKWELL PUBLISHING, INC, US LNKD- DOI:10.1002/JHET.5570210636, Bd. 21, Nr. 6, 1. November 1984 (1984-11-01), Seiten 1753-1754, XP002553839, ISSN: 0022-152X [gefunden am 2009-04-03] in der Anmeldung erwähnt

## Beschreibung

Die vorliegende Erfindung betrifft ein Verfahren zur Herstellung von 4-Amino-but-2-enoliden ausgehend von 4-Alkoxyfuran-2(5H)-on oder 4-Arylalkoxyfuran-2(5H)-on.

Bestimmte substituierte 4-Aminobut-2-enolid-Verbindungen sind als insektizid wirksame Verbindungen aus EP-A-0 539 588 und WO 2007/115644 bekannt. Sie können nach verschiedenen Methoden dargestellt werden.

So ist beispielsweise in Heterocycles Vol. 27, Nr. 8, Seite 1907 bis 1923 (1988) und in EP-A-0 539 588 beschrieben, dass Enaminocarbonylverbindungen (3) aus wasserfreier Tetronsäure (1) und einem Amin (2), wie in Schema 1 dargestellt, hergestellt werden können. Dieses Verfahren ist jedoch zur großtechnischen Herstellung von Enaminocarbonylverbindungen nicht gut geeignet, da die wasserfreie Tetronsäure (1) nicht kostengünstig herstellbar ist.

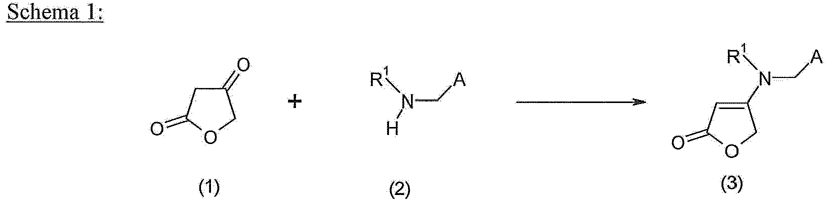

Bislang ist die Tetronsäure in größeren Mengen nicht käuflich erhältlich, so dass sie ausgehend von Acetessigester über Bromierung und anschließender Hydrierung (vgl. Synthetic Communication, 11(5), Seiten 385 bis 390 (1981)) zur Verwendung im oben beschriebenen Verfahren hergestellt werden muss. Die verhältnismäßig geringe Ausbeute an Tetronsäure (gewöhnlicherweise kleiner als 40%) und die Bedingung, dass Tetronsäure wasserfrei sein muss, verursacht hohe Kosten.

Ein weiteres Verfahren zur Herstellung von Tetronsäure ist in CH-PS 503 722 beschrieben. 4-Chloracetessigester wird mit einem aromatischen Amin zum 3-Arylaminocrotonlacton umgesetzt, wobei nach anschließender Behandlung mit Mineralsäure die Tetronsäure freigesetzt wird. Die Tetronsäure kann nur durch Hochvakmundestillation isoliert werden, was für den großtechnischen Einsatz dieses Verfahrens nachteilig ist.

Gleichfalls beschreibt die EP-A-0 153 615 ein mehrstufiges Verfahren zur Herstellung von Tetronsäure das von 2,4-Dichloracetessigestern ausgeht, das für die großtechnische Herstellung schlecht geeignet ist. Dieses Verfahren benötigt viele aufwändige Stufen und liefert die gewünschte Tetronsäure in einer vergleichsweise mäßigen Ausbeute von 65 %.

Ein weiteres Verfahren zur Herstellung von Tetronsäure ausgehend von Malonestern und Chloracetylchlorid ist aus J. Chem. Soc. Perkin Trans. 1 (1972), 9/10, S. 1225-1231 bekannt. Dieses Verfahren liefert die gewünschte Zielverbindung, jedoch mit einer Ausbeute von nur 43 %.

In Tetrahedron Letters, Nr. 31, Seiten 2683 und 2684 (1974) ist unter anderem die Herstellung von Tetronsäure, die in Schema 2 wiedergegeben ist, beschrieben. Als Edukt wird dabei Acetylendicarbonsäuredimethylester eingesetzt.

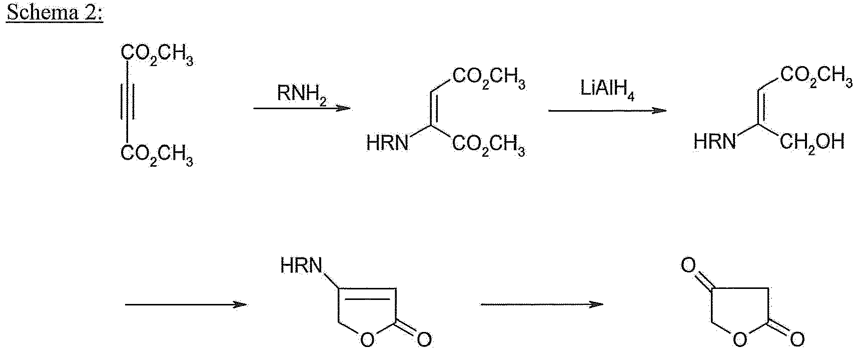

Nachteilig an diesem Verfahren ist die geringe Gesamtausbeute von nur 30 % sowie der Einsatz teurer Ausgangsstoffe, wie beispielsweise Lithiumaluminiumhydrid (LiAlH₄).

In WO 2007/115644 wird die Herstellung spezieller 4-Aminobut-2-enoliden beschrieben. Beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(5H)-on mit 3-Brom-1,1-dichlorprop-1-en oder durch Umsetzung von 4-[[2-Fluorethyl)amino]furan-2(5H)-on mit 2-Chlor-5-chlormethyl-pyridin. Die Reaktionen werden vorzugsweise mit Hydriden des Lithiums oder Natriums durchgeführt. Diese Substrate sind auch hier teuer und aus Sicherheitsgründen nur schwer zu handhaben.

Ein weiteres Verfahren zur Herstellung von substituierten 4-Aminobut-2-enoliden ist von Mowafak. et al. in J. Heterocyclic Chem., 21, 1753-1754 (1984) beschrieben. Dieses Verfahren geht von Methyltetronat aus, wobei durch Umsetzung mit Aminen die gewünschten Verbindungen hergestellt werden. Der darin beschriebene Austausch der Methoxygruppe durch eine "Michael Addition" lässt sich jedoch nur mit elektronenreichen cyclischen oder primären Aminen durchführen, was durch das Beipiel im experimentellen Teil dieser Anmeldung belegt ist.

Ausgehend von diesem Stand der Technik stellt sich somit die Aufgabe, ein Verfahren zur Herstellung von 4-Aminobut-2-enolid-Verbindungen der Formel (I) bereitzustellen, das die 4-Aminobut-2-enolid-Verbindungen mit hoher Ausbeute und ausreichend hoher Reinheit liefert und welches einfach und kostengünstig durchzuführen ist. Zusätzlich soll in dem Verfahren ein kostengünstig herzustellendes und einfach zu handhabendes Tetronsäurederivat verwendet werden.

Es wurde nun ein Verfahren zur Herstellung von 4-Aminobut-2-enolid-Verbindungen gefunden, das die vorgenannten Nachteile vermeidet und das einfach und kostengünstig durchführbar ist, insbesondere weil die erfindungsgemäßen 4-Aminobut-2-enolid-Verbindungen mit guten Ausbeuten und in hoher Reinheit erhalten werden, so dass eine aufwändige Aufarbeitung bzw. Reinigung des unmittelbaren Reaktionsprodukts gewöhnlicherweise nicht erforderlich ist.

Gegenstand der Erfindung ist somit das unten beschriebene Verfahren zur Herstellung von 4-Aminobut-2-enolid-Verbindungen der Formel (I): 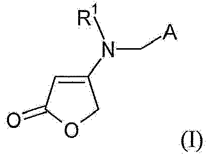 worin R¹ und A für die weiter unten definierten chemischen Gruppierungen stehen.

Das erfindungsgemäße Verfahren zur Herstellung von 4-Aminobut-2-enolid-Verbindungen der Formel (I) umfasst das Umsetzen einer 4-Alkoxyfuran-2(5H)-on-Verbindung oder 4-Arylalkoxyfiu-an-2(5H)-on der Formel (II) 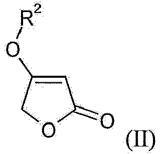 mit einem Amin der Formel (III) 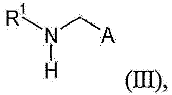 worin
- R¹: für Wasserstoff, C₁₋₁₂-Alkyl, C₁₋₁₂-Halogenalkyl, C₂₋₁₂-Alkei-iyl, C₂₋₁₂-Halogenalkenyl, C₂₋₆-Alkenyl, C₃₋₈-Cycloalkyl, C₃₋₈-CycloalkylC₁₋₆-alkyl, C₃₋₈-Halogencycloalkyl, C₁₋₁₂-Alkoxy, C₁₋₆-AlkyloxyC₁₋₆-alkyl, C₃₋₈-HalogencycloalkylC₁₋₆-alkyl oder ArylC₁₋₆-alkyl steht, bevorzugt steht R¹ für C₁₋₆-Alkyl, C₁₋₆-Halogenalkyl, C₂₋₆-Alkenyl, C₂₋₆-Halogenalkenyl, C₂₋₆-Alkinyl, C₃₋₈-Cycloalkyl, C₃₋₈-CycloalkylC₁₋₆-alkyl, C₃₋₈-Halogencycloalkyl, C₃₋₈-HalogencycloallcylC₁₋₆-alkyl oder C₁₋₆-AlkyloxyC₁₋₆-alkyl, besonders bevorzugt für Methyl, Ethyl, Propyl, Propylen, Propylen, Vinyl, Allyl, Propargyl, Cyclopropyl, C₁₋₆-AlkyloxyC₁₋₆-alkyl, 2-Fluorethyl, 2,2-Difluorethyl oder 2-Fluorcyclopropyl, ganz besonders bevorzugt für Methyl, Ethyl, n-Propyl, n-Prop-2-enyl, n-Prop-2-inyl, Cyclopropyl, Methoxyethyl, 2-Fluorethyl oder 2,2-Difluorethyl;
- R²: für C₁₋₁₂-Alkyl, ArylC₁₋₆-alkyl steht, bevorzugt steht R² für C₁-C₆-Alkyl, ArylC₁₋₆-alkyl, besonders bevorzugt für Benzyl, Methyl oder Ethyl; und
- A: für Pyrid-2-yl, Pyrid-4-yl oder Pyrid-3-yl steht, welche gegebenenfalls in 6-Position durch F, Cl, Br, CH₃, CF₃, oder OCF₃ substituiert sind, für Pyridazin-3-yl steht, welches gegebenenfalls in 6-Position durch Cl oder CH₃ substituiert ist, für Pyrazin-3-yl, 2-Chlor-pyrazin-5-yl oder für gegebenenfalls in 2-Position durch Cl oder CH₃ substituiertes 1,3-Thiazol-5-yl, für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, das gegebenenfalls durch F, Cl, Br, CN, NO₂, C₁₋₄-Alkyl, C₁₋₃-Alkylthio C₁₋₃-Alkylsulfonyl substituiert ist wobei jeder der Reste C₁₋₄-Alkyl, C₁₋₃-Alkylthio und C₁₋₃-Alkylsulfonyl durch F und/oder Chlor substituiert sein kann, oder für ein substituiertes Heterocyclyl der folgenden Formel steht 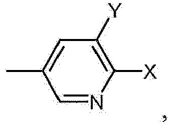
in der
- X: für Halogen, C₁₋₁₂-Alkyl oder C₁₋₁₂-Halogenalkyl steht und
- Y: für Halogen, C₁₋₁₂-Alkyl, C₁₋₁₂-Halogenalkyl, C₁₋₁₂-Halogenalkoxy, Azido oder CN steht; bevorzugt steht A für ein substituiertes Heterocyclyl ausgewählt unter 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3 -yl, 5-Me-thyl-6-iod-pyrid-3-yl, 5-Difluormethyl-6-fluor-pyrid-3-yl, 5-Difluormethyl-6-chlor-pyrid-3-yl, 5-Difluormetlryl-6-brom-pyrid-3-yl oder 5-Difluormethyl-6-iod-pyrid-3-yl, besonders bevorzugt steht A für ein substituiertes Heterocyclyl ausgewählt unter 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl oder 5-Difluormethyl-6-chlor-pyrid-3-yl, ganz besonders bevorzugt steht A für ein substituiertes Heterocyclyl ausgewählt unter 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl,

in Gegenwart einer Brønstedt-Säure, wobei die Brønstedt-Säure ausgewählt ist aus der Gruppe bestehend aus Phosphorsäure, Schwefelsäure, Salzsäure, Bromwasserstoffsäure, Flusssäure, Kaliumhydrogensulfat, Trifluoressigsäure, Methansulfonsäure und p-Toluolsulfonsäure und wobei das molare Verhätnis von Brønstedt-Säure zum Amin der Formel (III) im Bereich von 10:0,6 - 1:1,5 liegt.

Überraschenderweise wurde gefunden, dass in der erfindungsgemäßen Reaktion bzw. Umsetzung der Austausch des Alkoxyrestes in der Verbindung der Formel (II) gegen das Amin der Formel (III) in sehr guten Ausbeuten erfolgt. In Abwesenheit einer Brønstedt-Säure erfolgt keine Umsetzung, wie im Vergleichsbeispiel demonstriert ist. Weiterhin ist überraschend, dass die Tetronsäure unter den Reaktionsbedingungen nicht dimerisiert, obwohl eine solche Dimerisierung nach J. Chem. Soc. (1947) Seite 1365 zu erwarten war.

Die erfindungsgemäße Umsetzung kann in Gegenwart von Lösungsmitteln (Verdünnungsmitteln) durchgeführt werden. Das Lösungsmittel wird vorzugsweise in einer solchen Menge eingesetzt, dass das Reaktionsgemisch während des ganzen Verfahrens gut rührbar bleibt. Als Lösungsmittel zur Durchführung des erfindungsgemäßen Verfahrens bzw. Umsetzung kommen alle unter den Reaktionsbedingungen inerten organischen Lösungsmittel in Frage. Unter Lösungsmittel werden erfindungsgemäß auch Gemische reiner Lösungsmittel verstanden.

Erfindungsgemäß geeignete Lösungsmittel sind insbesondere Halogenkohlenwasserstoffe, wie Chlorkohlenwasserstoffe (z.B. Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Ether (z.B. Ethylpropylether, Methyl-tert-butylether, *n*-Butylether, Anisol, Phenetol, Cyclohexylmethylether, Dimethylether, Diethylether, Dimethylglycol Diphenylether, Dipropylether, Diisopropylether, Di-*n*-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether, und Polyether des Ethylenoxids und/oder Propylenoxids, Nitrokohlenwasserstoffe (z.B. Nitromethan, Nitroethan, Nitropropan, Nitrobenzol, Chlornitrobenzol, o-Nitrotoluol), Nitrile (z.B. Acetonitril, Methylnitril, Propionitril, Butyronitril, Isobutyronitril, Benzonitril, m-Chlorbenzonitril) sowie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Tetramethylensulfon, aliphatische, cycloaliphatische oder aromatische Kohlenwasserstoffe (z.B. Pentan, n-Hexan, n-Heptan, n-Oktan, Nonan sogenannte "White Spirits" mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat), Amide (z.B. Hexamethylenphosphorsäuretriamid, Formamid, N,N-Dimethylacetamid, *N*-Methyl-formamid, *N,N*-Dimethyl-formamid, *N,N*-Dipropyl-formamid, *N,N*-Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N*'-1,4-Diformyl-piperazin), und aliphatische Alkohole (z.B. Methanol, Ethanol, n-Propanol und iso-Propanol und n-Butanol) oder Gemische davon.

Für das erfindungsgemäße Verfahren bzw. die Umsetzung werden als Lösungsmittel bevorzugt Dioxan, Butyronitril, Propionitril, Acetonitril, Butylacetat, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon verwendet.

In Abhängigkeit der verwendeten Ausgangsverbindungen kann das erfindungsgemäße Verfahren bzw. die Umsetzung in Substanz, d.h. ohne Zusatz von Lösungsmitteln, durchgeführt werden.

Geeignete Brønstedt-Säuren sind grundsätzlich alle organischen und anorganischen Säuren. Erfindungsgemäß eingesetzte Brønstedt-Säuren sind Phosphorsäure (H₃PO₄), Schwefelsäure (H₂SO₄), Salzsäure (HCl), Bromwasserstoffsäure (HBr), Flusssäure (HF), Kaliumhydrogensulfat (KHSO₄), Trifluoressigsäure, Methansulfonsäure und p-Toluolsulfonsäure. Erfindungsgemäß besonders bevorzugt sind Phosphorsäure, Schwefelsäure, Kaliumhydrogensulfat und Trifluoressigsäure.

Die Brønstedt-Säure kann sowohl in wasserfreier als auch in wasserhaltiger Form, beispielsweise als 85%-ige Phosphorsäure oder 37-%ige Salzsäure, vorliegen. Aus wirtschaftlichen Gründen ist es bevorzugt, die im Handel erhältlichen Säurekonzentration zu verwenden.

Das molare Verhältnis der eingesetzten Brønstedt-Säure zu dem Amin der Formel (III) kann variieren. Erfindungsgemäß liegt das molare Verhältnis von Brønstedt-Säure zum Amin der Formel (III) im Bereich von 10 : 0,6 bis 1: 1,5, insbesondere von 5: 0,9 bis 1: 1,2, speziell von 2:1 bis 1: 1,1.

Das erfindungsgemäßen Verfahren kann im Allgemeinen im Vakuum, bei Normaldruck oder unter Überdruck durchgeführt werden.

Die angewendeten Temperaturen können in Abhängigkeit der verwendeten Ausgangsstoffe variieren. Die erfindungsgemäße Umsetzung bzw. das Verfahren kann bei Temperaturen im Bereich von etwa 20° bis etwa 200 °C, vorzugsweise bei Temperaturen im Bereich von etwa 20 °C bis etwa 150 °C durchgeführt werden.

Die Stöchiometrie der eingesetzten Verbindungen der Formel (II) und (III) kann in weiten Bereichen variieren. Das molare Verhältnis der Verbindung der Formel (II) zu dem eingesetzten Amin der Formel (III) kann etwa 1:0,5 bis etwa 1:10, insbesondere etwa 1:1 bis etwa 1:6, speziell etwa 1:1,05 bis etwa 1:2 betragen. Der Einsatz größerer Mengen an Verbindung der Formel (III) ist grundsätzlich möglich, jedoch aus wirtschaftlichen Gründen nachteilig.

Falls die Umsetzung in einem Lösungsmittel durchgeführt wird, kann das Lösungsmittel nach dem Reaktionsende durch Abdestillieren entfernt werden. Dieses kann unter Normaldruck oder erniedrigtem Druck bei Raumtemperatur oder erhöhten Temperaturen erfolgen.

Nach Ende der Reaktion kann die Brønstedt -Säure durch Extraktion mit Wasser entfernt werden. Die Isolierung der gewünschten Verbindungen der Formel (I) kann durch übliche Methoden erfolgen.

4-Methoxyfuran-2(5H)-on-Derivate der Formel (II) sind zum Teil bekannt und/oder können nach gängigen Methoden hergestellt werden.

Die Herstellung von Verbindung der Formel (II) in der R² für Methyl steht, ist beispielsweise in J. Heterocyclic Chem. 21, 1753 (1984) beschrieben. Weitere Herstellungen von Verbindungen der Formel (II) sind beispielsweise in European Journal of Organic Chemistry (2004), (22), 4582-4595 beschrieben.

Wenn nicht anders definiert, wird unter dem Begriff "Alkyl", entweder in Alleinstellung oder aber in Kombination mit weiteren Begriffen, wie beispielsweise Halogenalkyl, Alkoxyalkyl, Cycloalkylalkyl, Halogencycloalkylalkyl und Arylalkyl, wird im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁₋₁₂-Alkylreste sind Methyl, Ethyl, n-Propyl, isoPropyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alyklresten sind C₁₋₆-Alkylreste besonders bevorzugt. Insbesondere bevorzugt sind C₁₋₄-Alkylreste, speziell Methyl und Ethyl.

Wenn nicht anders definiert, wird unter dem Begriff "Alkenyl" wird erfindungsgemäß ein linearer oder verzweigter C₂₋₁₂-Alkenylrest, welcher mindestens eine Doppelbindung aufweist, beispielsweise Vinyl, Allyl, 1-Propoenyl, Isopropenyl, 1-Butenyl, 2-Butenyl, 3-Butenyl, 1,3-Butandienyl, 1-Pentenyl, 2-Pentenyl, 3-Pentenyl, 4-Pentenyl, 1,3-Pentandienyl, 1-Hexenyl, 2-Hexenyl, 3-Hexenyl, 4-Hexenyl, 5-Hexenyl und 1,4-Hexandienyl, verstanden. Bevorzugt hiervon sind C₂₋₆-Alkenylreste und besonders bevorzugt sind C₂₋₄-Alkenylreste.

Wenn nicht anders definiert, wird unter Unter dem Begriff "Alkinyl" wird erfindungsgemäß ein linearer oder verzweigter C₂₋₁₂-Alkinylrest, welcher mindestens eine Dreifachbindung aufweist, beispielsweise Ethinyl, 1-Propinyl und Propargyl, verstanden. Bevorzugt hiervon sind C₂₋₆-Alkinylreste und besonders bevorzugt sind C₃₋₄-Alkinylreste. Der Alkinylrest kann dabei auch mindestens eine Doppelbindung aufweisen.

Wenn nicht anders definiert, wird unter Unter dem Begriff "Cycloalkyl" wird erfindungsgemäß ein C₃₋₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃₋₆-Cycloalkylreste.

Wenn nicht anders definiert, wird unter Unter dem Begriff "Aryl" wird erfindungsgemäß ein aromatischer Rest mit 6 bis 14 Kohlenstoffatomen, vorzugsweise Phenyl, verstanden.

Wenn nicht anders definiert, wird unter dem Begriff "Arylalkyl" eine Kombination von erfindungsgemäß definierten Resten "Aryl" und "Alkyl" verstanden, wobei der Rest über die Alkylgrupe gebunden wird, Beispiele hierfür sind Benzyl, Phenylethyl oder α-Methylbenzyl, wobei Benzyl besonders bevorzugt ist.

Wenn nicht anders definiert, werden "durch Halogen substituierte Reste", beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Wenn nicht anders definiert, wird unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die vorgenannte Bedeutung aufweist.

Gegebenenfalls substituierte Reste können einfach oder mehrfach substituiert sein, wobei bei einer Mehrfachsubstitution die Substituenten gleich oder verschieden sein können.

Die vorliegende Erfindung wird anhand der nachfolgenden Beispiele näher erläutert, ohne die Erfindung auf diese einzuschränken.

### Herstellungsbeispiele:

### Vergleichsbeispiel (analog wie in J. Heterocyclic Chem., 21, 1753-1754 (1984) beschrieben)

0,3 g (2,6 mmol) 4-Methoxyfuran-2(5H)-on und 0,54 g (2,6 mmol) 5 g N-[(6-Chlorpyridin-3-yl)methyl)-2,2-difluorethylamin werden in 3 ml Dioxan vorgelegt und 15 h unter Rückfluß erhitzt. Es erfolgt keine Umsetzung.

### Erfindungsgemäßes Beispiel

0,3 g (2,6 mmol) 4-Methoxyfuran-2(5H)-on und 0,54 g (2,6 mmol) N-[(6-Chlorpyridin-3-yl)methyl)-2,2-difluorethylamin werden in 3 ml Dioxan vorgelegt und mit 1 ml 32 %iger Salzsäure versetzt. Anschließend wird 8 h unter Rückfluß erhitzt. Die Reaktionsmischung wird auf Wasser gegossen und 2 mal mit Methylenchlorid extrahiert. Die Methylenchlorid-Phase wird getrocknet über Natriumsulfat und anschließend das Lösungsmittel im Vakuum entfernt. Man erhält 0,6 g 4-[[(6-Chlorpyridin-3yl)methyl](2,2-difluorethyl)amino]furan-2(5H)-on (79 % Ausbeute).

¹R-NMR (CDCl₃, 298K) δ: 3,53 (td, 2H), 4,52 (s, 2H), 4,82 (s, 2H), 4,83 (s, 1H), 5,96 (tt, 1H), 7,37 (d, 1H), 7,55 (dd, 1H), 8,27( d, 1H)

## Patentansprüche

1. Verfahren zur Herstellung von 4-Aminobut-2-enolid-Verbindungen der Formel (I) 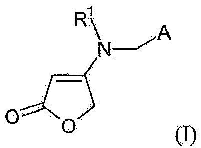 umfassend das Umsetzen einer 4-Alkoxyfuran-2(5H)-on Verbindung oder 4-Arylalkoxyfuran-2(5H)-on Verbindung der Formel (II) 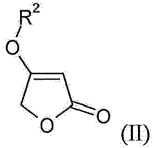 mit einem Amin der Formel (III) 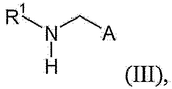 worin
R¹ für Wasserstoff, C₁₋₁₂-Alkyl, C₁₋₁₂-Halogenalkyl, C₂₋₁₂-Alkenyl, C₁₋₁₂-Halogenalkenyl, C₂₋₆-Alkinyl, C₃₋₈-Cycloalkyl, C₃₋₈-CycloalkylC₁₋₆-alkyl, C₃₋₈-Halogencycloalkyl, C₁₋₁₂-Alkoxy, C₁₋₆-AlkyloxyC₁₋₆-alkyl, C₃₋₈-HalogencycloalkylC₁₋₆-alkyl oder AlylC₁₋₆-alkyl steht;
R² für C₁₋₁₂-Alkyl, oder ArylC₁₋₆-alkyl steht, wobei unter Aryl ein aromatischer Rest mit 6 bis 14 Kohlenstoffatomen zu verstehen ist; und
A für Pyrid-2-yl, Pyrid-4-yl oder Pyrid-3-yl steht, welches gegebenenfalls in 6-Position durch F, Cl, Br, CH₃, CF₃, oder OCF₃ substituiert ist, für Pyridazin-3-yl steht, welches gegebenenfalls in 6-Position durch Cl oder CH₃ substituiert ist, für Pyrazin-3-yl, 2-Chlor-pyrazin-5-yl oder für gegebenenfalls in 2-Position durch Cl oder CH₃ substituiertes 1,3-Thiazol-5-yl, für Pyrimidinyl, Pyrazolyl, Thiophenyl, Oxazolyl, Isoxazolyl, 1,2,4-Oxa-diazolyl, Isothiazolyl, 1,2,4-Triazolyl oder 1,2,5-Thiadiazolyl steht, das gegebenenfalls durch F, Cl, Br, CN, NO₂, C₁₋₄-Alkyl, C₁₋₃-Alkylthio C₁₋₃-Alkylsulfonyl substituiert ist wobei jeder der Reste C₁₋₄-Alkyl, C₁₋₃-Alkylthio und C₁₋₃-Alkylsulfonyl durch F und/oder Chlor substituiert sein können, oder für ein substituiertes Heterocyclyl der folgenden Formel steht 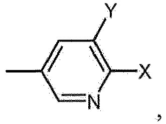
in der
X für Halogen, C₁₋₁₂-Alkyl oder C₁₋₁₂-Halogenalkyl steht, und
Y für Halogen, C₁₋₁₂-Alkyl, C₁₋₁₂-Halogenalkyl, C₁₋₁₂-Halogenalkoxy, Azido oder CN steht;
in Gegenwart einer Brønstedt-Säure, wobei die Brønstedt-Säure ausgewählt ist aus der Gruppe bestehend aus Phosphorsäure, Schwefelsäure, Salzsäure, Bromwasserstoffsäure, Flusssäure, Kaliumhydrogensulfat, Trifluoressigsäure, Methansulfonsäure und p-Toluolsulfonsäure und wobei das molare Verhätnis von Brønstedt-Säure zum Amin der Formel (III) im Bereich von 10:0,6 - 1:1,5 liegt.

2. Verfahren nach Anspruch 1, worin in Verbindung (III)
R¹ für C₁₋₆-Alkyl, C₁₋₆-Halogenalkyl, C₂₋₆-Alkenyl, C₂₋₆-Halogenalkenyl, C₂₋₆-Alkinyl, C₃₋₈-Cycloalkyl, C₃₋₈-CycloalkylC₁₋₆-alkyl, C₃₋₈-Halogencycloalkyl, C₃₋₈-HalogeticycloalkylC₁₋₆-alkyl oder C₁₋₆-AlkyloxyC₁₋₆-alkyl steht; und
A ausgewählt ist unter 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluonnethyl-6-fluor-pyrid-3-yl, 5-Difluormethyl-6-chlor-pyrid-3-yl, 5-Difluormethyl-6-brom-pyrid-3-yl oder 5-Difluormethyl-6-iod-pyrid-3-yl.

3. Verfahren nach einem der Ansprüche 1 bis 2, worin das molare Verhältnis der Verbindung der Formel (II) zu dem eingesetzten Amin der Formel (III) 1: 0,5 bis 1: 10 beträgt.

4. Verfahren nach einem der Ansprüche 1 bis 3, worin das molare Verhältnis der eingesetzten Brønstedt-Säure zu dem Amin der Formel (III) im Bereich von 5 : 0,8 bis 1: 1,5 liegt.

## Claims

1. Process for the preparation of 4-aminobut-2-enolide compounds of the formula (I) 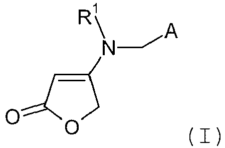 comprising the reaction of a 4-alkoxyfuran-2(5H)-one compound or 4-arylalkoxyfuran-2(5H)-one compound of the formula (II) 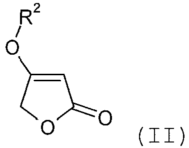 with an amine of the formula (III) 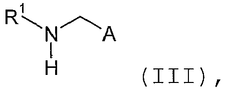 in which
R¹ is hydrogen, C₁₋₁₂-alkyl, C₁₋₁₂-haloalkyl, C₂₋₁₂-alkenyl, C₂₋₁₂-haloalkenyl, C₂₋₆-alkynyl, C₃₋₈-cycloalkyl, C₃₋₈-cycloalkyl-C₁₋₆-alkyl, C₃₋₈-halocycloalkyl, C₁₋₁₂-alkoxy, C₁₋₆-alkyloxy-C₁₋₆-alkyl, C₃₋₈-halocycloalkyl-C₁₋₆-alkyl or aryl-C₁₋₆-alkyl ;
R² is C₁₋₁₂-alkyl, or aryl-C₁₋₆-alkyl, where aryl is to be understood as meaning an aromatic radical having 6 to 14 carbon atoms; and
A is pyrid-2-yl, pyrid-4-yl or pyrid-3-yl, which is optionally substituted in the 6-position by F, Cl, Br, CH₃, CF₃, or OCF₃, is pyridazin-3-yl, which is optionally substituted in the 6-position by Cl or CH₃, is pyrazin-3-yl, 2-chloropyrazin-5-yl or is 1,3-thiazol-5-yl optionally substituted in the 2-position by Cl or CH₃, is pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl, which is optionally substituted by F, Cl, Br, CN, NO₂, C₁₋₄-alkyl, C₁₋₃-alkylthio C₁₋₃-alkylsulphonyl, where each of the radicals C₁₋₄-alkyl, C₁₋₃-alkylthio and C₁₋₃-alkylsulphonyl may be substituted by F and/or chlorine, or is a substituted heterocyclyl of the following formula 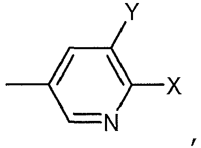
in which
X is halogen, C₁₋₁₂-alkyl or C₁₋₁₂-haloalkyl, and
Y is halogen, C₁₋₁₂-alkyl, C₁₋₁₂-haloalkyl, C₁₋₁₂-haloalkoxy, azido or CN;
in the presence of a Brønsted acid, wherein the Brønsted acid is selected from the group consisting of phosphoric acid, sulphuric acid, hydrochloric acid, hydrobromic acid, hydrofluoric acid, potassium hydrogensulphate, trifluoroacetic acid, methanesulphonic acid and p-toluenesulphonic acid, and wherein the molar ratio of Brønsted acid to the amine of the formula (III) is in the range of 10:0.6-1:1.5.

2. Process according to Claim 1, wherein, in compound (III),
R¹ is C₁₋₆-alkyl, C₁₋₆-haloalkyl, C₂₋₆-alkenyl, C₂₋₆-haloalkenyl, C₂₋₆-alkynyl, C₃₋₈-cycloalkyl, C₃₋₈-cycloalkyl-C₁₋₆-alkyl, C₃₋₈-halocycloalkyl, C₃₋₈- halocycloalkyl-C₁₋₆-alkyl or C₁₋₆-alkyloxy-C₁₋₆-alkyl; and
A is selected from 6-fluoropyrid-3-yl, 6-chloropyrid-3-yl, 6-bromopyrid-3-yl, 6-methylpyrid-3-yl, 6-trifluoromethylpyrid-3-yl, 6-trifluoromethoxypyrid-3-yl, 6-chloro-1,4-pyridazin-3-yl, 6-methyl-1,4-pyridazin-3-yl, 2-chloro-1,3-thiazol-5-yl or 2-methyl-1,3-thiazol-5-yl, 2-chloropyrimidin-5-yl, 2-trifluoromethylpyrimidin-5-yl, 5,6-difluoropyrid-3-yl, 5-chloro-6-fluoropyrid-3-yl, 5-bromo-6-fluoropyrid-3-yl, 5-iodo-6-fluoropyrid-3-yl, 5-fluoro-6-chloropyrid-3-yl, 5,6-dichloropyrid-3-yl, 5-bromo-6-chloropyrid-3-yl, 5-iodo-6-chloropyrid-3-yl, 5-fluoro-6-bromopyrid-3-yl, 5-chloro-6-bromopyrid-3-yl, 5,6-dibromopyrid-3-yl, 5-fluoro-6-iodopyrid-3-yl, 5-chloro-6-iodopyrid-3-yl, 5-bromo-6-iodopyrid-3-yl, 5-methyl-6-fluoropyrid-3-yl, 5-methyl-6-chloropyrid-3-yl, 5-methyl-6-bromopyrid-3-yl, 5-methyl-6-iodopyrid-3-yl, 5-difluoromethyl-6-fluoropyrid-3-yl, 5-difluoromethyl-6-chloropyrid-3-yl, 5-difluoromethyl-6-bromopyrid-3-yl or 5-difluoromethyl-6-iodopyrid-3-yl.

3. Process according to one of Claims 1 to 2, wherein the molar ratio of the compound of the formula (II) to the amine of the formula (III) used is 1:0.5 to 1:10.

4. Process according to one of Claims 1 to 3, wherein the molar ratio of the Brønsted acid used to the amine of the formula (III) is in the range from 5:0.8 to 1:1.5.

## Revendications

1. Procédé de fabrication de composés de 4-aminobut-2-énolide de formule (I) 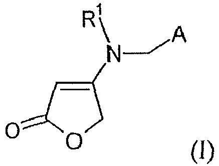 comprenant la mise en réaction d'un composé de 4-alcoxyfuran-2(5H)-one ou d'un composé de 4-arylalcoxyfuran-2(5H)-one de formule (II) 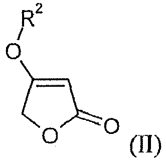 avec une amine de formule (III) 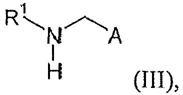 dans lesquelles
R¹ représente hydrogène, alkyle en C₁₋₁₂, halogénoalkyle en C₁₋₁₂, alcényle en C₂₋₁₂, halogénoalcényle en C₂₋₁₂, alcynyle en C₂₋₆, cycloalkyle en C₃₋₈, cycloalkyle en C₃₋₈-alkyle en C₁₋₆, halogénocycloalkyle en C₃₋₈, alcoxy en C₁₋₁₂, alkyloxy en C₁₋₆-alkyle en C₁₋₆, halogénocycloalkyle en C₃₋₈-alkyle en C₁₋₆ ou aryl-alkyle en C₁₋₆ ;
R² représente alkyle en C₁₋₁₂ ou aryl-alkyle en C₁₋₆, aryle signifiant un radical aromatique de 6 à 14 atomes de carbone ; et
A représente pyrid-2-yle, pyrid-4-yle ou pyrid-3-yle, qui est éventuellement substitué en position 6 par F, Cl, Br, CH₃, CF₃ ou OCF₃ ; pyridazin-3-yle, qui est éventuellement substitué en position 6 par Cl ou CH₃ ; pyrazin-3-yle, 2-chloro-pyrazine-5-yle ou 1,3-thiazol-5-yle éventuellement substitué en position 2 par Cl ou CH₃ ; pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par F, Cl, Br, CN, NO₂, alkyle en C₁₋₄, alkylthio en C₁₋₃, alkylsulfonyle en C₁₋₃, chacun des radicaux alkyle en C₁₋₄, alkylthio en C₁₋₃ et alkylsulfonyle en C₁₋₃ pouvant être substitué par F et/ou chlore ; ou un hétérocyclyle substitué de la formule suivante : 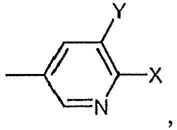 dans laquelle
X représente halogène, alkyle en C₁₋₁₂ ou halogénoalkyle en C₁₋₁₂, et
Y représente halogène, alkyle en C₁₋₁₂, halogénoalkyle en C₁₋₁₂, halogénoalcoxy en C₁₋₁₂, azido ou CN ;
en présence d'un acide de Bronsted, l'acide de Bronsted étant choisi dans le groupe constitué par l'acide phosphorique, l'acide sulfurique, l'acide chlorhydrique, le bromure d'hydrogène, l'acide fluorhydrique, l'hydrogénosulfate de potassium, l'acide trifluoroacétique, l'acide méthanesulfonique et l'acide p-toluènesulfonique, et le rapport molaire entre l'acide de Bronsted et l'amine de formule (III) se situant dans la plage allant de 10:0,6 à 1:1,5.

2. Procédé selon la revendication 1, dans lequel, dans le composé (III),
R¹ représente alkyle en C₁₋₆, halogénoalkyle en C₁₋₆, alcényle en C₁₋₆, halogénoalcényle en C₂₋₆, alcynyle en C₂₋₆, cycloalkyle en C₃₋₈, cycloalkyle en C₃₋₈-alkyle en C₁₋₆, halogénocycloalkyle en C₃₋₈, halogénocycloalkyle en C₃₋₈-alkyle en C₁₋₆ ou alkyloxy en C₁₋₆-alkyle en C₁₋₆ ; et
A est choisi parmi 6-fluoro-pyrid-3-yle, 6-chloro-pyrid-3-yle, 6-bromo-pyrid-3-yle, 6-méthyl-pyrid-3-yle, 6-trifluorométhyl-pyrid-3-yle, 6-trifluorométhoxy-pyrid-3-yle, 6-chloro-1,4-pyridazin-3-yle, 6-méthyl-1,4-pyridazin-3-yle, 2-chloro-1,3-thiazol-5-yle ou 2-méthyl-1,3-thiazol-5-yle, 2-chloro-pyrimidin-5-yle, 2-trifluorométhyl-pyrimidin-5-yle, 5,6-difluoro-pyrid-3-yle, 5-chloro-6-fluoro-pyrid-3-yle, 5-bromo-6-fluoro-pyrid-3-yle, 5-iodo-6-fluoro-pyrid-3-yle, 5-fluoro-6-chloro-pyrid-3-yle, 5,6-dichloro-pyrid-3-yle, 5-bromo-6-chloro-pyrid-3-yle, 5-iodo-6-chloro-pyrid-3-yle, 5-fluoro-6-bromo-pyrid-3-yle, 5-chloro-6-bromo-pyrid-3-yle, 5,6-dibromo-pyrid-3-yle, 5-fluoro-6-iodo-pyrid-3-yle, 5-chloro-6-iodo-pyrid-3-yle, 5-bromo-6-iodo-pyrid-3-yle, 5-méthyl-6-fluoro-pyrid-3-yle, 5-méthyl-6-chloro-pyrid-3-yle, 5-méthyl-6-bromo-pyrid-3-yle, 5-méthyl-6-iodo-pyrid-3-yle, 5-difluorométhyl-6-fluoro-pyrid-3-yle, 5-difluorométhyl-6-chloro-pyrid-3-yle, 5-difluorométhyl-6-bromo-pyrid-3-yle ou 5-difluorométhyl-6-iodo-pyrid-3-yle.

3. Procédé selon l'une quelconque des revendications 1 à 2, dans lequel le rapport molaire entre le composé de formule (II) et l'amine de formule (III) utilisée est de 1:0,5 à 1:10.

4. Procédé selon l'une quelconque des revendications 1 à 3, dans lequel le rapport molaire entre l'acide de Bronsted utilisé et l'amine de formule (III) est dans la plage allant de 5:0,8 à 1:1,5.
